# EUROPEAN PATENT SPECIFICATION

(11) **EP 2 417 923 B1**
(45) Date of publication and mention of the grant of the patent: **13.11.2013**
(21) Application number: 11176512.9
(22) Date of filing: 04.08.2011
(51) Int. Cl.: A61B 17/66

(54) **External fixator system**
Externes Befestigungssystem
Système de fixateur externe

(30) Priority: 11.08.2010 EP 10172523
(43) Date of publication of application: 15.02.2012
(73) Proprietor: Stryker Trauma SA, 2545 Selzach (CH)
(72) Inventor: Singh, Manoj Kumar, 122 001 Harayana (IN); Crozet, Yves, Ramsey, New Jersey 07446 (US); Burgherr, Vinzenz, 3084 Wabern (CH)
(74) Representative: Frischknecht, Harry Ralph

(56) References cited:
- EP-A2- 1 136 041
- US-A- 5 885 282
- US-A1- 2004 059 331

## Description

### TECHNICAL FIELD

The present invention relates to an external fixator according to claim 1.

### PRIOR ART

Currently, there are many bone deformities that external fixators can correct using the distraction osteogenesis process. The Ilizarov device (or similar system) is widely used. This system uses rings also designated as fixation plates connected by threaded rods or struts with nuts to manipulate angulations, translation and length discrepancies of bones. The nuts are manually adjusted by the patient with a wrench or by hand to move rings and/or percutaneous fixation components. As the position adjustments of the components must be done where the nuts are secured, it can be difficult for the patient to make the required daily adjustments with consideration of stable fixation in mind. Other devices use different techniques to adjust the effective length of the rods but all must be adjusted somewhere between the ends - offering limited access for the patient. As the adjustments are often a daily task for the patient, easier access to the frame adjustment points would be a significant advantage.

In particular it is a need for a patient to receive a feedback concerning the quantity of the actuation in order to gain knowledge about the adjustment for each of the struts.

Furthermore the current design of the connection between the strut and the fixation plates are not very ergonomic concerning actuation by the user.

US 2004/0059331 shows an external fixator device that allows different manipulation.

Additionally prior art devices have also the disadvantage that in case the user applies a rather high force rotational movement of the strut with regard to the ring can occur. This is negative with regard to the precision of the length of the struts in particular in case the patient adjusts the length of the strut.

### SUMMARY OF THE INVENTION

It is therefore an object of the present invention to provide an external fixator having an actuation unit which overcomes the drawback of prior art devices. Furthermore it is an object of the present invention to provide a device which can be actuated with a greater accuracy.

This object is solved by an external fixator according to claim 1. Such an external fixator comprising at least two fixation plates which are arranged at a distance to each another, at least one length-adjustable strut having a first end region and a second end region which are in connection with said fixation plates and at least one actuation unit to adjust the length of said strut(s) in order to adjust the distance and/or orientation between said fixation plates. Said actuation unit is in a fixed connection with at least one of said fixation plates as well as with at least one of the end regions of the respective strut in order to connect the strut with said fixation plate. The actuation unit comprises an outer sleeve with an opening and an actuation element which is arranged rotatable in said opening. Preferably said actuation element extends fully through said opening. Said actuation element is connected to said end region of said strut and said actuation unit is connected to said plates via parts of said outer sleeve. Said outer sleeve extends into an opening which is arranged in said fixation plate. The opening and the outer sleeve have a corresponding shape such that a relative rotation between the outer sleeve and the fixation plate is prevented. Therefore the device can be actuated with a greater accuracy as there is no rotational movement permitted.

Preferably said outer sleeve comprises a clamping section and a bearing section, wherein said clamping section comprises a threaded section and a shaft section and wherein the bearing section comprises said corresponding shape preventing relative rotation.

At least parts of said clamping section and at least parts of said bearing section are encompassed by said opening which results in an advantageous mechanical structure.

Preferably the outer sleeve comprises a flange preferably arranged between the clamping section and the bearing section and wherein said opening preferably comprises a shoulder, wherein said flange and said shoulder are in contact with each other, when the outer sleeve is inserted into said opening.

The bearing section has a rectangular cross section and wherein the opening has a corresponding rectangular cross section, which rectangular cross sections prevents relative rotation.

Preferably the actuation unit comprises a socket which is adapted to receive a tool to be actuated, whereby the socket is oriented such that it extends in or almost in direction of said strut and/or such that it extends perpendicular to a front surface of the fixation plate. This orientation has the advantage that the user gains very ergonomic access to actuate the actuation element. Hence this mechanism allows the effective length to be adjusted from the top which allows easier patient access. This increases patient compliance - one of a significant challenges in deformity correction with an external fixator.

In a further embodiment the device shall provide the user with a feedback concerning the quantity of the actuation and it shall be provided in a very compact manner. Furthermore said actuation unit comprises a feedback unit which provides the user with a feedback concerning the degree of the actuation.

The actuation unit provides a gradual telescopic motion due to the arrangement of a feedback unit which is advantageous for the patient/user. Due to the fixed connection with the fixation plate it is possible to provide a very compact structure as the actuation unit can be arranged partly within said plate.

Preferably the feedback unit provides the user with a haptic and/or an acoustic and/or a visual feedback while the actuation unit is actuated.

Preferably the feedback unit comprises a spring-loaded ball which engages in one of a plurality of corresponding chambers such that upon a relative movement between the spring-loaded ball and the corresponding chambers said ball moves from its originating chamber to a neighbouring chamber.

Preferably the feedback unit is arranged in connection with the outer sleeve and the actuation element.

Preferably the spring-loaded ball is arranged in an opening within the actuation element and wherein the chambers are arranged within the through opening of the outer sleeve, or the spring-loaded ball is arranged in an opening within the outer sleeve and wherein the chambers are arranged within the actuation element.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1a to 1c: show a schematic view of an external fixator which is here arranged to support the foot of a patient;
- Fig. 2: shows an actuation unit to be used in the external fixator of figure 1;
- Fig. 3: shows a cross-sectional view of the actuation unit of figure 2;
- Fig. 4: shows a perspective sectional view of the actuation unit of figure 3;
- Fig. 5: shows the actuation unit of the previous figures in connection with an length-adjusting strut to be used to connect two rings of the external fixator with each other; and
- Fig. 6: shows a detailed view of figure 5.

### DESCRIPTION OF PREFERRED EMBODIMENTS

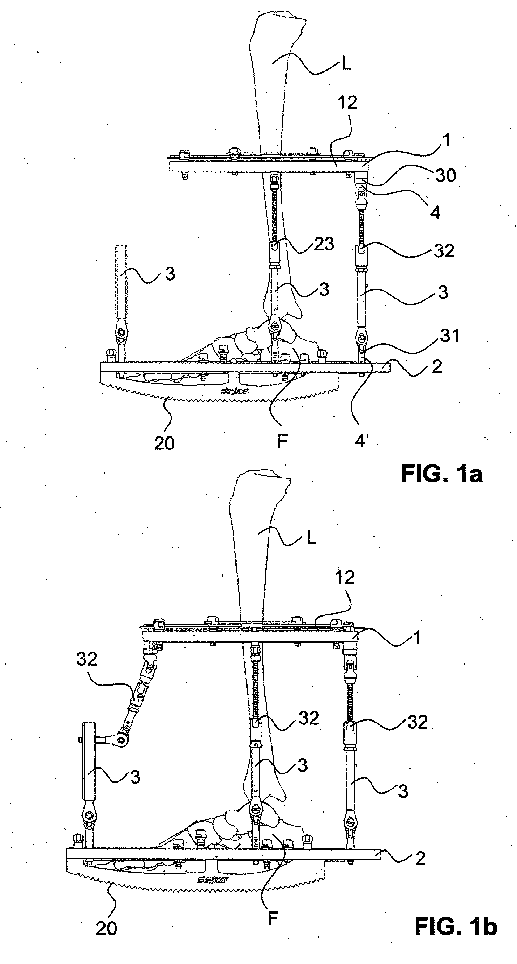

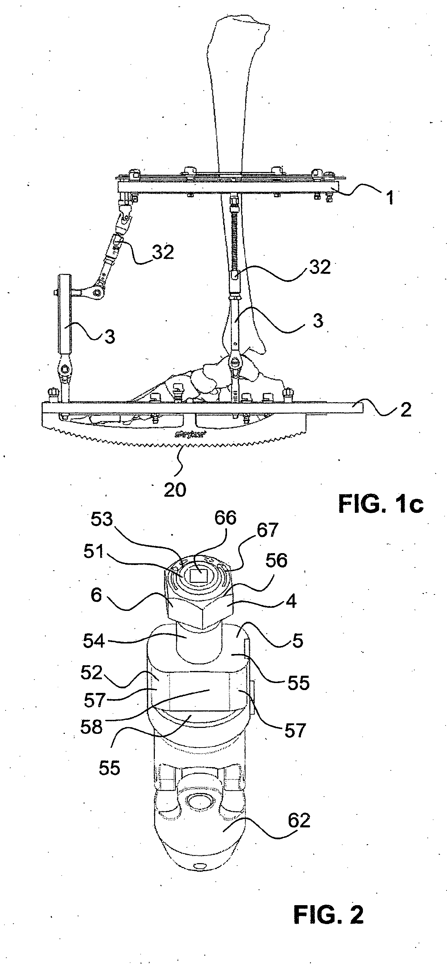

Figures 1a to 1c show an exemplary embodiment of an external fixator. The external fixator comprises at least two fixation plates 1, 2 which are arranged at a distance to each other and at least one length-adjusting strut 3 which is in connection with the fixation plates 1, 2. The fixation plates 1, 2 serve as bearing elements for pins which are in connection with bony structure. Thereby the orientation as well as the distance between two fixation plates 1, 2 defines the orientation and distance between fractured elements of said bony structure. Each of the fixation plate 1, 2 comprises also a front surface 12 which extends over the largest dimension of the plate 1, 2

In the present embodiment there is an upper fixation plate 1 in connection with the lower leg L and a lower fixation plate 2 in connection with the foot F. The lower fixation plate 2 comprises also a rolling structure 20 to enable a user to walk around.

The struts 3 mainly comprise a length-adjusting mechanism 32 here in the shape of a threaded strut 33 and a non-rotating strut 34 with a thread in which the threaded strut 33 engages. Via its first end region 30 and its second end region 31 the struts 3 are in connection with the respective fixation plate. In the present embodiment the struts 3 are connected to the upper fixation plate 1 by means of an actuation unit 4 and to the lower fixation plate 2 by means of a clamping element 4'. It is also possible to use an actuation unit 4 to connect the strut 3 to the upper fixation plate 1 as well as to the lower fixation plate 2. The actuation unit 4 is provided to actuate the length-adjusting strut in order to change its length.

The actuation unit 4 is in a fixed connection with the fixation plate 1, 2. The term fixed connection is to be understood as being a connection which prevents unintentional relative motion between the actuation unit 4 and the fixation plate 1, 2. In particular a rotative motion shall be prevented. Preferably the fixation plates 1, 2 comprise a plurality of openings 10 in which such actuation units 4 can be arranged and the fixed connection can be established. The fixed connection has the advantage that device can be adjusted very easily without the aid of several tools.

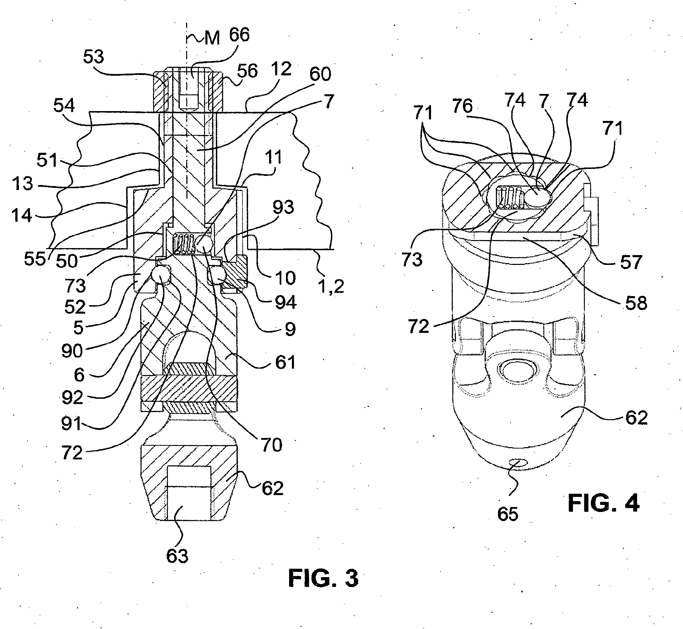

Figure 2 shows the actuation unit 4 in a perspective view and figures 3 and 4 show sectional views. The actuation unit 4 comprises an outer sleeve 5 in which an actuation element 6 is arranged. The actuation unit 4 is in connection with the fixation plate 1, 2 by means of the outer sleeve 5.

The outer sleeve 5 extends along a middle axis M and comprises a through opening 50, a clamping section 51 and a bearing section 52.

The clamping section 51 comprises a threaded section 53 and a shaft section 54. The threaded section is in contact with a nut 56 which is used to secure the outer sleeve 5 to the fixation plate 1, 2. On the outer side a flange 55 divides the clamping section 51 from the bearing section 52. The bearing section 52 has mainly two purposes, namely to bear the actuation element and to provide a bearing of the outer sleeve 5 in the opening 10. Hence the inner side provided by said through opening 50 serves to provide a bearing for the actuation element 6. The outer side of the bearing section 52 serves mainly to provide a bearing for the outer sleeve 5 within said opening 10 in the ring 1 as explained below with regard to figure 3 in more detail. The outer side of the bearing section 52 has in the present embodiment a rectangular cross-section with rounded edges 57 and flat sidewalls 58. Edges 57 and sidewalls 58 extend parallel to the middle axis M. The part which is located in vicinity of flange 55 however, is also in connection with the opening in the fixation plate 1, 2.

In figure 3 the opening 10 in the fixation plate 1, 2 is schematically shown. The opening 10 comprises a shoulder 11 which subdivides the opening 10. The opening 10 comprises a first section 13 and a second section 14. The shoulder 11 is located between the first section 13 and the second section 14. The first section 13 of the opening 10 has therefore a complementary or corresponding shape as the shaft section 54. In the present embodiment shaft section 54 as well as first section 13 have circular cross-sections and the second section 14 as well as the bearing section 52 have a rectangular cross-section.

When the outer sleeve 5 is inserted into the opening 10 the shoulder 11 is in contact with the flange 55. The shaft section 54 of the outer sleeve 5 extends through the first section 13 of the opening 10 and the bearing section 52 extends into the second section 14. The outer sleeve 5 is fixed to the fixation plate 1, 2 by means of the nut 56 which retracts the outer sleeve 55 relative the fixation plate 1, 2 such that the flange 55 comes in contact with the shoulder 11.

From figure 2 it becomes evident that the cross-section of the outer surface of the bearing section 52 which is in contact with the opening 10 is provided such that the a rotation of the outer sleeve 5 relative to the fixation plate 1, 2 is prevented. For that reason the opining 10 has a complementary shape. In the present embodiment the outer sleeve 5 has partly a rectangular cross-section with rounded edges. Here the rectangular cross-section is mainly provided by the outer side of the bearing section 52 or the outer surfaces of the bearing section 52, respectively.

The actuation element 6 extends also along the middle axis M and comprises mainly a shaft section 60 which extends through the opening 50 of the outer sleeve and a connection section 61 which is in connection with the strut 3. The actuation element 6 can be actuated, i.e. rotated, by means of a tool 67 (as shown in figure 7) which engages in a socket 66 of the actuation element 6. Socket 66 is thereby arranged such that the tool can be introduced in a direction which is more or less in line with the axis of the strut or in a direction perpendicular to the fixation plate 1, 2, in particular to the surface 12. The orientation of the socket 66 has thereby the advantage that easy access is provided from the top of the fixation device and that the length of the struts 3 can be adjusted very easily.

The actuation element 6 is borne by means of a ball bearing 9 in the outer sleeve 5. In the present embodiment the ball bearing 9 is provided by means of the shaft section 61 and the bearing section 52. A separate ball bearing is also possible, but a ball bearing which is provided according to the embodiment of figure 3 is very compact in terms of size.

The bearing section 52 and the shaft section 61 comprises respective grooves 90, 91 in which a plurality of balls 92 are arranged. Groove 90 extends into the surface of the opening 50 and encompasses the whole opening 50, whereas groove 91 is arranged in the shaft 61 of the actuation element 6. The grooves 90, 91 provide a channel in which the balls 92 are arranged. Balls 92 may be introduced into said channel via an opening 93 in the shaft section 61 which is covered by means of a cover 94.

Between the outer sleeve 5 and the actuation element 6 there is arranged a feedback unit 7. The feedback unit 7 is best seen in figure 3 and 4. In the present embodiment the feedback unit 7 is provided by means of a spring-loaded ball 70 and corresponding chambers 71. The spring-loaded ball 70 is arranged in an opening 72 in the actuation element 6. Between the ground of the opening 72 and the spring-loaded ball 70 there is arranged a spring 73 which provides a force that pushes the ball 70 to the respective chamber 71. The chambers 71 are arranged in the surface of the through opening 50 in the outer sleeve 5. Upon rotation of the actuation element 6 relative to the outer sleeve 5 the spring-loaded ball 70 will pushed against the spring force by means of the transition portion 74 between two neighbouring chambers 71. As soon as the next chamber 71 is in line with the spring axis, the spring-loaded ball 70 will be moved into the respective chamber 71. This mechanism results in a clicking noise which provides the user with a respective feedback about the amount of his actuation.

There are a plurality of chambers 71 arranged which are distributed evenly around the perimeter of the through opening 50 of the outer sleeve 5. In the present embodiments eight chambers 71 are arranged but it is also possible to arrange more or less than eight chambers. The number of chambers depends on the application.

With regard to the embodiment as shown in figure 3 and 4 it has to be noted that the opening 72 can also be arranged in the outer sleeve 5 and that the chambers 71 can also be arranged in the actuation element. With such a configuration the same result can be achieved.

The strut 3 with its end region is in a fixed connection with the actuation element 6. In the present embodiment there is a cardan joint 62 arranged between the strut 3 and the actuation element 6 in order to compensate angular differences between the strut 3 and the actuation element 6. Furthermore the actuation element 6 comprises an opening 63 in which the strut 3 extends. Preferably the strut 3 is in connection with said opening 63 by means of a thread, a press fit or any other suitable connection method which prevents a relative movement between the strut 3 and the actuation element 6. In case a thread is used it is advantageous to secure the thread by means of a pin 64 which extends through the opening 63 and the strut 3. For that reason a pin opening 65 is arranged in the region of the opening 63.

The use of a cardan joint 62 has the advantage that the adjustments can be made in advantageous manner, namely in a more precise and a very smooth manner.

Upon rotation of the actuation element 6 the strut 3 will also be rotated and its length will be adjusted according to the degree of rotation. The feedback unit 7 then provides the user with an acoustic as well as with a haptic feedback due to its mechanical structure as outlined above.

The arrangement of the feedback unit 7 as mentioned herein has the advantage that in terms of dimension a very compact structure can be achieved. Thereby the overall-weight can be significantly reduced and it is more convenient for the patient to use such a structure.

In figure 2 it can also be recognized that markings 67 concerning the direction of rotation are arranged in order to allow the user to know in which direction the actuation element 4 shall be actuated. In this region it is also possible to arrange a scale on which the user can visually recognize the amount of rotation, whereby a visual feedback can be provided.

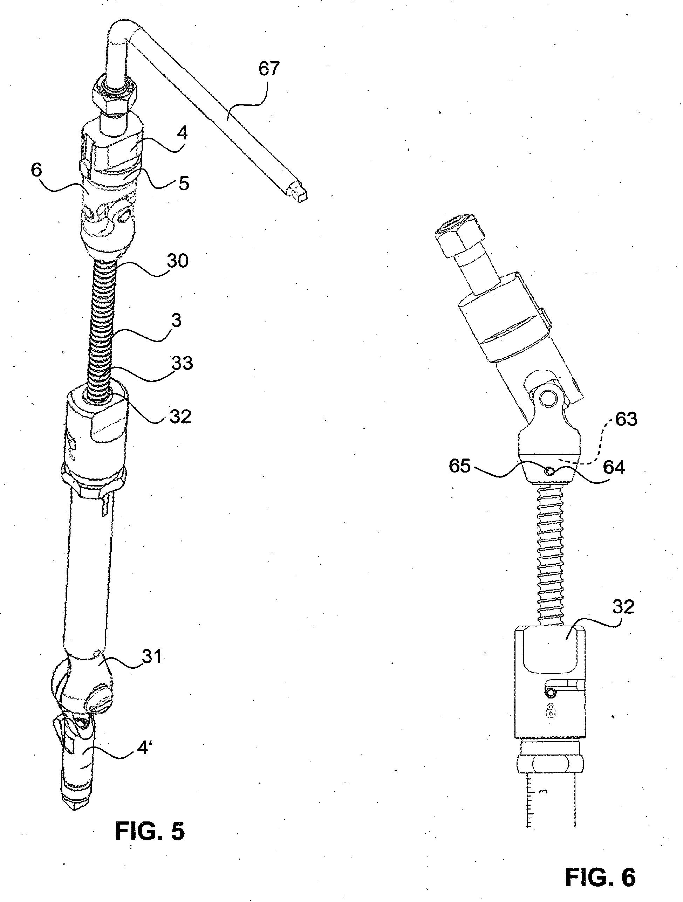

Figures 5 and 6 show the strut 3 in connection with actuation unit 4 via its first end region 31 and with the clamping element 4' via its second end region 32. The clamping element 4' clamps the strut 3 in fixed manner to the fixation plate 1, 2 which is not shown here. The actuation unit 4 is also in a fixed connection with the respective fixation plate, but the actuation element 6 which is arranged within the actuation unit 5 is rotateable relative to the actuation unit. A rotation of the actuation element results in a rotation of the threaded strut 33 and in connection with the non-rotating strut section 34 the length of the strut 3 will be adjusted.

### LIST OF REFERENCE SIGNS

- 1: fixation plate
- 2: fixation plate
- 3: strut
- 4: actuation unit
- 4': clamping element
- 5: outer sleeve
- 6: actuation element
- 7: feedback unit
- 8: actuation element
- 9: ball bearing

- 10: opening
- 11: shoulder
- 12: front surface
- 13: first section
- 14: second section
- 20: rolling structure

- 30: first end region
- 31: second end region
- 32: length adjusting mechanism
- 33: threaded section
- 34: non-rotating strut section

- 50: through opening
- 51: clamping section
- 52: bearing section
- 53: threaded section
- 54: shaft section

- 55: flange
- 56: nut
- 57: edges
- 58: sidewalls

- 60: shaft section
- 61: connection section
- 62: cardan joint
- 63: opening
- 64: pin
- 65: pin opening
- 66: socket
- 67: tool

- 70: spring-loaded ball
- 71: chambers
- 72: opening
- 73: spring
- 74: transition portion

- 90: groove
- 91: groove
- 92: ball
- 93: opening
- 94: cover

- L: lower leg
- F: foot
- M: middle axis

## Claims

1. External fixator comprising at least two fixation plates (1, 2) which are arranged at a distance to each another, at least one length-adjustable strut (3) having a first end region (30) and a second end region (31) which are in connection with said fixation plates (1, 2) and at least one actuation unit (4) to adjust the length of said strut(s) (3) in order to adjust the distance and/or orientation between said fixation plates (1, 2), wherein said actuation unit (4) is in a fixed connection with at least one of said fixation plates (1, 2) as well as with at least one of the end regions (30, 31) of the respective strut (3) in order to connect the strut (3) with said fixation plate (1, 2) wherein the actuation unit (4) comprises an outer sleeve (5) with an opening (50) and an actuation element (6) which is arranged rotatable in said opening (50), wherein said actuation element (6) is in a fixed connection connected to said end region of said strut, wherein, upon rotation of the actuation element (6) the end region (30) of the strut (3) connected with the actuation unit (4) will also be rotated and the length of the strut will be adjusted according to the degree of rotation, wherein said actuation unit (4) is connected to said plates (1, 2) via parts of said outer sleeve (5) and said outer sleeve (5) extends into an opening (10) which is arranged in said fixation plate (1, 2), wherein the opening (10) and the outer sleeve (5) have a corresponding shape such that a relative rotation between the outer sleeve (5) and the fixation plate (1, 2) is prevented.

2. External fixator according to claim 1, wherein the outer sleeve (5) comprises a clamping section (51) and a bearing section (52), wherein said clamping section comprises a threaded section (53) and a shaft section (54) and wherein the bearing section (52) comprises said corresponding shape preventing relative rotation.

3. External fixator according to claim 2, wherein at least parts of said clamping section (51) and at least parts of said bearing section (52) are encompassed by said opening (10).

4. External fixator according to any of claims 2 to 3, wherein the outer sleeve (5) comprises a flange (55) preferably arranged between the clamping section (51) and the bearing section (52) and wherein said opening (10) comprises a shoulder (11), wherein said flange (55) and said shoulder (11) are in contact with each other, when the outer sleeve (5) is inserted into said opening (10).

5. External fixator according to any of claims 2 to 4, wherein the bearing section (52) has a rectangular cross section and wherein the opening (10) has a corresponding rectangular cross section, which rectangular cross sections prevents relative rotation.

6. External fixator according to any of the preceding claims, wherein the actuation unit (4) comprises a socket (66) which is adapted to receive a tool (67) to be actuated, whereby the socket (66) is oriented such that it extends in or almost in direction of said strut and/or such that it extends perpendicular to a front surface (12) of the fixation plate (1, 2).

7. External fixator according to any of the preceding claims, wherein said actuation unit (4) comprises a feedback unit (7) which provides the user with a feedback concerning the degree of the actuation.

8. External fixator according to claim 7, **characterized in that** the feedback unit (5) provides the user with a haptic and/or an acoustic and/or a visual feedback while the actuation unit (4) is actuated.

9. External fixator according to claims 7 to 8, **characterized in that** the feedback unit comprises a spring-loaded ball (70) which engages in one of a plurality of corresponding chambers (71) such that upon a relative movement between the spring-loaded ball (70) and the corresponding chambers (71) said ball (70) moves from its originating chamber to a neighbouring chamber (71).

10. External fixator according to claims 7 to 9, **characterized in that** the feedback unit (7) is arranged in connection with the outer sleeve (5) and the actuation element (6).

11. External fixator according to claims 7 to 10, **characterized in that** the spring-loaded ball (70) is arranged in an opening (72) within the actuation element (6) and wherein the chambers (71) are arranged within the through opening (50) the outer sleeve (5), or **in that** the spring-loaded ball (70) is arranged in an opening (72) within the outer sleeve (5) and wherein the chambers (71) are arranged within the actuation element (6).

12. External fixator according to claims 7 to 11, **characterized in that** chambers are distributed in a regular spacing around the perimeter of the through opening (50) of the outer sleeve (5) or around the actuation element (5).

## Patentansprüche

1. Externer Fixateur umfassend mindestens zwei Fixierplatten (1, 2), welche mit einer Distanz zueinander angeordnet sind, mindestens ein längenveränderbarer Stab (3) mit einem ersten Endbereich (30) und einem zweiten Endbereich (31), welche mit der besagten Fixierplatten (1,2) in Verbindung sind, und mindestens eine Aktuatoreinheit (4), um die Länge der besagten Stäbe bzw. des besagten Stabes (3) einzustellen, um die Distanz und/oder Orientierung zwischen den besagten Fixierplatten (1, 2) einzustellen, wobei besagte Aktuatoreinheit (4) in einer festen Verbindung mit mindestens einer der besagten Fixierplatten (1, 2) sowie mit mindestens einer der besagten Endbereiche (30, 31) der entsprechenden Stäbe (3) ist, um den Stab (3) mit der besagten Fixierplatte (1, 2) zu verbinden, wobei die Aktuatoreinheit (4) eine äussere Hülse (5) mit einer Öffnung (50) und ein Aktuatorelement (6) umfasst, welches rotierbar in der besagten Öffnung (50) angeordnet ist, wobei besagtes Aktuatorelement (6) in einer festen Verbindung mit dem besagten Endbereich des besagten Stabes verbunden ist, wobei bei Rotation des Aktuatorelementes (6) der Endbereich (30) des Stabes (3), der mit der Aktuatoreinheit (4) in Verbindung steht, auch rotiert wird und die Länge des Stabes gemäss dem Grad der Rotation eingestellt wird, wobei besagte Aktuatoreinheit (4) zu den besagten Platten (1, 2) über Teile der besagten äusseren Hülse (5) befestigt ist und wobei besagte äussere Hülse (5) in eine Öffnung (10), welche in der besagten Fixierplatte (1, 2) angeordnet ist, einragt, wobei die Öffnung (10) und die äussere Hülse (5) eine korrespondierende Form haben, sodass relative Rotation zwischen der äusseren Hülse (5) und der Fixierplatte (1, 2) verhindert wird.

2. Externer Fixateur gemäss Anspruch 1, wobei die äussere Hülse (5) einen Klemmabschnitt (51) und einen Lagerabschnitt (52) umfasst, wobei besagter Klemmabschnitt einen Gewindeabschnitt (53) und einen Schaftabschnitt (54) umfasst, und wobei der Lagerabschnitt (52) die besagte korrespondierende Form, welche die relative Rotation verhindert, umfasst.

3. Externer Fixateur nach Anspruch 2, wobei mindestens Teile des besagten Klemmabschnittes (51) und mindestens Teile des besagten Lagerungsabschnittes (52) durch die besagte Öffnung (10) umfasst sind.

4. Externer Fixateur nach einem der Ansprüche 2 bis 3, wobei die äussere Hülse (5) einen Flansch (55) umfasst, der vorzugsweise zwischen dem Klemmabschnitt (51) und dem Lagerabschnitt (52) angeordnet ist, und wobei besagte Öffnung (10) eine Schulter (11) umfasst, wobei besagter Flansch (55) und besagte Schulter (11) in Kontakt miteinander sind, wenn die äussere Hülse (5) in die besagte Öffnung (10) eingeschoben ist.

5. Externer Fixateur gemäss einem der Ansprüche 2 bis 4, wobei der Lagerungsabschnitt (52) einen rechteckigen Querschnitte aufweist, und wobei die Öffnung (10) einen korrespondierenden rechteckigen Querschnitt hat, welche rechteckigen Querschnitte die relative Rotation verhindern.

6. Externer Fixateur gemäss einer der vorhergehenden Ansprüche, wobei die Aktuatoreinheit (4) eine Aufnahme (66) umfasst, welche geeignet ist, ein zu betätigendes Werkzeug (67) aufzunehmen, wobei die Aufnahme (66) so orientiert ist, dass sich diese in oder fast in Richtung des besagten Stabes und/oder dass sie sich rechtwinklig zu einer vorderen Oberfläche (12) der Fixierplatte (1, 2) erstreckt.

7. Externer Fixateur gemäss einem der vorhergehenden Ansprüche, wobei besagte Aktuatoreinheit (4) eine Rückmeldeeinheit (7) umfasst, welche dem Benutzer eine Rückmeldung bezüglich dem Grad der Betätigung bereitstellt.

8. Externer Fixateur gemäss Anspruch 7, **dadurch gekennzeichnet, dass** die Rückmeldeeinheit (5) dem Benutzer eine haptische und/oder eine akustische und/oder eine visuelle Rückmeldung während der Betätigung der Aktuatoreinheit (4) bereitstellt.

9. Externer Fixateur gemäss Ansprüche 7 bis 8, **dadurch gekennzeichnet, dass** der Rückmeldeeinheit (4) eine mit einer Feder vorgespannte Kugel (70) umfasst, welche in eine Mehrzahl von entsprechenden Kammern (71) eingreift, sodass aufgrund einer relativen Bewegung zwischen der federvorgespannten Kugel (70) und der entsprechenden Kammer (71) die besagte Kugel (70) von ihrer ursprünglichen Kammer in eine benachbarte Kammer (71) bewegt wird.

10. Externer Fixateur gemäss Ansprüchen 7 bis 9, **dadurch gekennzeichnet, dass** die Rückmeldeeinheit (7) in Verbindung mit der äusseren Hülse (5) und dem Aktuatorelement (6) angeordnet ist.

11. Externer Fixateur gemäss Ansprüchen 7 bis 10, **dadurch gekennzeichnet, dass** die federvorgespannte Kugel (70) in einer Öffnung (72) innerhalb des Aktuatorelementes (6) angeordnet ist, und wobei die Kammern (71) innerhalb der Durchgangsöffnung (50) der äusseren Hülse (5) angeordnet sind, oder dass die federvorgespannte Kugel (70) in einer Öffnung (72) innerhalb der äusseren Hülse (5) angeordnet ist und wobei die Kammern (71) innerhalb des Aktuatorelementes (6) angeordnet sind.

12. Externer Fixateur gemäss Ansprüchen 7 bis 11, **dadurch gekennzeichnet, dass** die Kammern über einen regelmässigen Abstand um den Umfang der Durchgangsöffnung (50) der äusseren Hülse (5) oder um das Aktuatorelement (5) angeordnet sind.

## Revendications

1. Fixateur externe comprenant au moins deux plaques de fixation (1, 2) qui sont disposées à distance l'une de l'autre, au moins une entretoise (3) de longueur ajustable ayant une première région d'extrémité (30) et une deuxième région d'extrémité (31) qui sont connectées auxdites plaques de fixation (1, 2) et au moins une unité d'actionnement (4) pour ajuster la longueur de ladite ou desdites entretoises (3) afin d'ajuster la distance et/ou l'orientation entre lesdites plaques de fixation (1, 2), ladite unité d'actionnement (4) étant connectée fixement à au moins l'une desdites plaques de fixation (1, 2), ainsi qu'à au moins l'une des régions d'extrémité (30, 31) de l'entretoise respective (3) afin de connecter l'entretoise (3) à ladite plaque de fixation (1, 2), l'unité d'actionnement (4) comprenant un manchon externe (5) avec une ouverture (50) et un élément d'actionnement (6) qui est agencé de manière rotative dans ladite ouverture (50), ledit élément d'actionnement (6) étant connecté fixement à ladite région d'extrémité de ladite entretoise, et lors de la rotation de l'élément d'actionnement (6), la région d'extrémité (30) de l'entretoise (3) connectée à l'unité d'actionnement (4) étant également tournée et la longueur de l'entretoise étant ajustée en fonction du degré de rotation, ladite unité d'actionnement (4) étant connectée auxdites plaques (1, 2) par le biais de parties dudit manchon externe (5) et ledit manchon externe (5) s'étendant dans une ouverture (10) qui est agencée dans ladite plaque de fixation (1, 2), l'ouverture (10) et le manchon externe (5) ayant une forme correspondante de telle sorte qu'une rotation relative entre le manchon externe (5) et la plaque de fixation (1, 2) soit empêchée.

2. Fixateur externe selon la revendication 1, dans lequel le manchon externe (5) comprend une section de serrage (51) et une section de support (52), ladite section de serrage comprenant une section filetée (53) et une section de tige (54) et la section de support (52) comprenant ladite forme correspondante empêchant la rotation relative.

3. Fixateur externe selon la revendication 2, dans lequel au moins des parties de ladite section de serrage (51) et au moins des parties de ladite section de support (52) sont englobées par ladite ouverture (10).

4. Fixateur externe selon l'une quelconque des revendications 2 à 3, dans lequel le manchon externe (5) comprend une bride (55) agencée de préférence entre la section de serrage (51) et la section de support (52) et dans lequel ladite ouverture (10) comprend un épaulement (11), ladite bride (55) et ledit épaulement (11) étant en contact l'un avec l'autre lorsque le manchon externe (5) est inséré dans ladite ouverture (10).

5. Fixateur externe selon l'une quelconque des revendications 2 à 4, dans lequel la section de support (52) présente une section transversale rectangulaire et dans lequel l'ouverture (10) a une section transversale rectangulaire correspondante, ces sections transversales rectangulaires empêchant la rotation relative.

6. Fixateur externe selon l'une quelconque des revendications précédentes, dans lequel l'unité d'actionnement (4) comprend une douille (66) qui est adaptée pour recevoir un outil (67) destiné à être actionné, la douille (66) étant orientée de telle sorte qu'elle s'étende dans la direction ou pratiquement dans la direction de ladite entretoise et/ou de telle sorte qu'elle s'étende perpendiculairement à une surface frontale (12) de la plaque de fixation (1, 2).

7. Fixateur externe selon l'une quelconque des revendications précédentes, dans lequel ladite unité d'actionnement (4) comprend une unité de réaction (7) qui fournit à l'utilisateur une réaction concernant le degré d'actionnement.

8. Fixateur externe selon la revendication 7, **caractérisé en ce que** l'unité de réaction (5) fournit à l'utilisateur une réaction tactile et/ou acoustique et/ou visuelle lorsque l'unité d'actionnement (4) est actionnée.

9. Fixateur externe selon les revendications 7 à 8, **caractérisé en ce que** l'unité de réaction comprend une bille (70) précontrainte par ressort qui s'engage dans l'une parmi une pluralité de chambres correspondantes (71) de telle sorte que lors d'un mouvement relatif entre la bille (70) précontrainte par ressort et les chambres correspondantes (71), ladite bille (70) se déplace de sa chambre d'origine jusqu'à une chambre adjacente (71).

10. Fixateur externe selon les revendications 7 à 9, **caractérisé en ce que** l'unité de réaction (7) est agencée de manière à être connectée au manchon externe (5) et à l'élément d'actionnement (6).

11. Fixateur externe selon les revendications 7 à 10, **caractérisé en ce que** la bille (70) précontrainte par ressort est agencée dans une ouverture (72) à l'intérieur de l'élément d'actionnement (6), les chambres (71) étant agencées à l'intérieur de l'ouverture traversante (50) du manchon externe (5), ou **en ce que** la bille (70) précontrainte par ressort est agencée dans une ouverture (72) à l'intérieur du manchon externe (5), les chambres (71) étant agencées à l'intérieur de l'élément d'actionnement (6).

12. Fixateur externe selon les revendications 7 à 11, **caractérisé en ce que** des chambres sont réparties suivant un espacement régulier autour du périmètre de l'ouverture traversante (50) du manchon externe (5) ou autour de l'élément d'actionnement (5).
